(19) Europäisches Patentamt
European Patent Office
Office européen des brevets

(11) Veröffentlichungsnummer: **0 173 824 B1**

# (12) EUROPÄISCHE PATENTSCHRIFT

(45) Veröffentlichungstag der Patentschrift: **08.11.89**

(51) Int. Cl.⁴: **C 07 D 461/00**

(21) Anmeldenummer: **85108625.6**

(22) Anmeldetag: **11.07.85**

(54) **Racemische oder optisch aktive 9- beziehungsweise 11-substituierte Apovincaminsäurederivate und Verfahren zu ihrer Herstellung.**

(30) Priorität: **11.07.84 HU 270284**

(43) Veröffentlichungstag der Anmeldung: **12.03.86 Patentblatt 86/11**

(45) Bekanntmachung des Hinweises auf die Patenterteilung: **08.11.89 Patentblatt 89/45**

(84) Benannte Vertragsstaaten: **AT BE CH DE FR GB IT LI NL SE**

(56) Entgegenhaltungen:
**FR-A- 2 320 302**
**FR-A- 2 341 585**
**FR-A- 2 342 980**

**CHEMICAL ABSTRACTS, Band 91, Nr. 9, 27. August 1979, Seite 617, Zusammenfassung Nr. 74767f, Columbus, Ohio, US; P. SARLET et al.: "Nitration of vincamine and vincamone - hemisynthesis of vincinone"**

(73) Patentinhaber: **Richter Gedeon Vegyészeti Gyár R.T., Gyömröi ut 19-21, H-1475 Budapest X (HU)**

(72) Erfinder: **Vedres, András, Dr. Dipl.-Chem., Füredi ut 56, H-1144 Budapest (HU)**
Erfinder: **Szántay, Csaba, Dr. Ing., Szombolyai u. 8, H-1113 Budapest (HU)**
Erfinder: **Moldvai, István, Kossuth L.u. 107, H-1212 Budapest (HU)**
Erfinder: **Stefkó, Béla, Dr. Dipl.-Chem., Orlay u. 2/b, H-1117 Budapest (HU)**

(74) Vertreter: **Beszédes, Stephan G., Dr. Patentanwalt, Münchener Strasse 80a Postfach 1168, D-8060 Dachau (DE)**

**EP 0 173 824 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue racemische oder optisch aktive 9- beziehungsweise 11-substituierte Apovincaminsäurederivate und ein Verfahren zu ihrer Herstellung.

Es ist kein Verfahren zum Nitrieren der Apovincaminsäure bekannt. Bekannt sind hingegen die 9- und 11-(Nitro)-derivate des Vincamines und des Apovincamines, deren Herstellung zum Beispiel in den französischen Patentschriften 2 341 585, 2 320 302 und 2 342 80 beschrieben ist. Sowohl im Falle des Vincamines als auch im Falle des Apovincamines wird in eisessigsaurem Medium mit Salpetersäure nitriert. Dabei entstehen Gemische von 9- und 11-(Nitro)-verbindungen. Bei Vincamin wird durch die Reaktion die Entstehung des 11-(Nitro)-vincamines begünstigt (Bull. Soc. Chim. Belg. 88 [1979], 1 bis 2), während im Falle des Apovincamines die Menge des gebildeten 9-(Nitro)-apovincamines überwiegt. Zur Beeinflussung des Verhältnisses der beiden Isomere wurde kein Verfahren festgestellt und die Trennung des erhaltenen Produktgemisches erfolgt umständlich auf chromatographischem Wege und ist mit beträchtlichen Substanzverlusten verbunden.

Auch ist es aus der französischen Patentschrift 2 342 980 bekannt, 9- und 11-(Nitro)-apovincamin zu 9- beziehungsweise 11-(Amino)-apovincamin zu reduzieren. In der französischen Patentschrift 2 342 980 ist zur Herstellung von 9- und 11-(Amino)-apovincamin die mit Zinkstaub in Gegenwart von Calciumchlorid vorgenommene Reduktion der entsprechenden Nitroverbindungen beschrieben, jedoch bringt diese Reaktion nur mittlere Ausbeuten. Das Studium der im Schrifttum beschriebenen Reaktionen ergab, daß die Reduktion der Nitroapovincaminisomere mittels katalytischen Hydrierens nur mit geringer Ausbeute zu den entsprechenden Aminoapovincaminisomeren führt, weil als Hauptprodukt durch Sättigung der 14,15-Doppelbindung das Dihydroderivat entsteht.

Der Erfindung liegt die Aufgabe zugrunde, neue Zwischenprodukte zur Herstellung von anderen therapeutisch wertvollen 9- oder 11-substituierten Apovincaminsäurederivaten, die leicht und einfach mit hoher Ausbeute herstellbar sind, sowie ein leicht und einfach mit hoher Ausbeute durchführbares Verfahren zur Herstellung der ersteren zu schaffen.

Das Obige wurde überraschenderweise durch die Erfindung erreicht.

Gegenstand der Erfindung sind racemische oder optisch aktive 9- beziehungsweise 11-substituierte Apovincaminsäurederivate der allgemeinen Formel

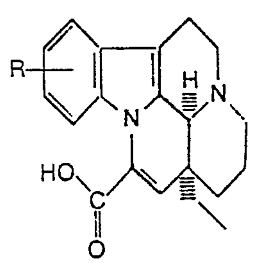

I

worin

R für eine Nitro- oder Aminogruppe in der 9- oder 11-Stellung steht,

sowie ihre Salze.

Von ihnen sind (+)-9-(Nitro)-apovincaminsäure, (+)-11-(Nitro)-apovincaminsäure, (−)-9-(Amino)-apovincaminsäure und (+)-11-Aminoapovincaminsäure sowie ihre Salze bevorzugt.

Die 9- beziehungsweise 11-(Nitro)-apovincaminsäure kann speziell durch die folgenden Formeln Ia beziehungsweise Ib dargestellt werden:

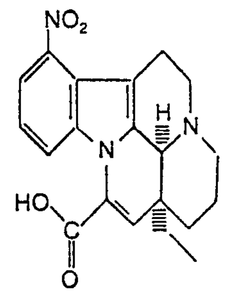

Ia

beziehungsweise

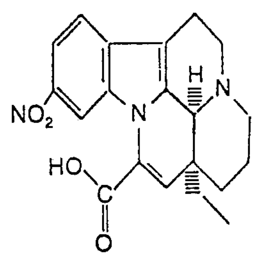

Ib.

Die aus ihnen durch Reduktion erhältlichen 9- beziehungsweise 11-(Amino)-apovincaminsäure entsprechen den folgenden Formeln Ic beziehungsweise Id.

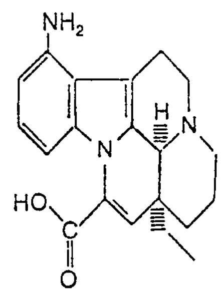

Ic

beziehungsweise

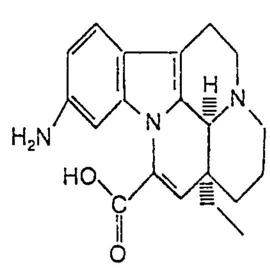

Id

Gegenstand der Erfindung ist auch ein Verfahren zur Herstellung der erfindungsgemäßen 9- beziehungsweise 11-substituierten Apovincaminsäurederivate, welches dadurch gekennzeichnet ist, daß Apovincaminsäure der Formel

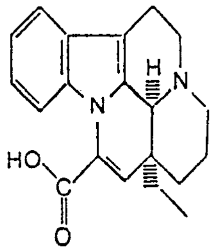

II

nitriert wird und gegebenenfalls die erhaltene(n) 9- und/oder 11-(Nitro)-apovincaminsäure(n), gegebenenfalls nach ihrem Isolieren aus dem Reaktionsgemisch, reduziert wird beziehungsweise werden sowie gegebenenfalls in an sich bekannter Weise die erhaltenen 9- beziehungsweise 11-substituierten Apovincaminsäurederivate der allgemeinen Formel I in Säureadditionssalze oder quaternäre Salze beziehungsweise Metall- beziehungsweise Ammoniumsalze überführt werden und/oder
die erhaltenen Salze der 9- beziehungsweise 11-substituierten Apovincaminsäurederivate der allgemeinen Formel I in die freien 9- beziehungsweise 11-substituierten Apovincaminsäurederivate der allgemeinen Formel I oder in andere Säureadditionssalze beziehungsweise quaternäre Salze beziehungsweise Metall- beziehungsweise Ammoniumsalze überführt werden und/oder gegebenenfalls eine Spaltung der erhaltenen racemischen 9- beziehungsweise 11-substituierten Apovincaminsäurederivate der allgemeinen Formel I beziehungsweise von deren Säureadditionssalzen beziehungsweise quaternären Salzen beziehungsweise Metall- beziehungsweise Ammoniumsalzen in ihre optischen Antipoden beziehungsweise eine Racemisierung der entsprechenden optisch aktiven Verbindungen in Stereoisomere vorgenommen wird.

Vorzugsweise wird das Nitrieren mit konzentrierter Salpetersäure in eisessigsaurem Medium durchgeführt.

Erfindungsgemäß wird also wie im genannten Stand der Technik vorzugsweise in eisessigsaurem Medium mit Salpetersäure nitriert. Überraschenderweise wurde jedoch festgestellt, daß durch zweckmäßige Wahl der Reaktionsbedingungen das Produktverhältnis [das Verhältnis der 9-(Nitro)-apovincaminsäure der Formel Ia zur 11-(Nitro)-apovincaminsäure der Formel Ib] innerhalb bestimmter Grenzen beeinflußt werden kann.

Wenn nämlich die Umsetzung in reinem Eisessig oder in 1 bis 3 Vol.-% Acetonitril beziehungsweise Dimethylformamid enthaltendem Eisessig vorgenommen wird, dann entstehen 9- und 11-(Nitro)-apovincaminsäure in etwa gleichen Mengen. Nach einer Ausführungsform des erfindungsgemäßen Verfahrens wird daher das Nitrieren in 1 bis 3 Vol.-% Acetonitril oder Dimethylformamid enthaltendem Eisessig durchgeführt. Als Variante davon kann auch in reinem Eisessig gearbeitet werden.

Wenn andererseits zum Nitrieren Eisessig, welcher 10 bis 50 Vol.-% Chloroform enthält, verwendet wird, verschiebt sich die Reaktion in Richtung der Bildung von 11-(Nitro)-apovincaminsäure. Nach einer anderen vorteilhaften Ausführungsform des erfindungsgemäßen Verfahrens wird also das Nitrieren in 10 bis 50 Vol.-% Chloroform enthaltendem Eisessig durchgeführt.

Darüberhinaus kann die Richtung des Nitrierens auch durch die Temperatur beeinflußt werden. Es wurde festgestellt, daß, obwohl die Umsetzung innerhalb eines weiten Temperaturbereiches, zweckmäßig von −15 bis +45 °C, vorgenommen werden kann, der Temperaturbereich von 0 bis +16 °C besonders zweckmäßig ist. Nach einer weiteren bevorzugten Ausführungsform des erfindungsgemäßen Verfahrens wird daher das Nitrieren bei Temperaturen von 0 bis +16 °C, insbesondere 0 bis +5 °C, durchgeführt. Wenn innerhalb dieses Temperaturbereiches an der unteren Grenze gearbeitet wird, dann entsteht überwiegend 11-(Nitro)-apovincaminsäure und die Menge der das Produkt verunreinigenden sonstigen Begleitstoffe bleibt unter 5%. In der Nähe der oberen Grenze des genannten Temperaturbereiches entstehen die beiden Isomeren in etwa gleichen Mengen und die Menge der schwer identifizierbaren Nebenprodukte steigt etwas an.

Bei Durchführung des Nitrierens bei Temperaturen von 0 bis 16 °C dauert die Reaktion etwa 2 Stunden.

Zweckmäßig wird das Isolieren der 9- und/oder 11-(Nitro)-apovincaminsäure(n) in Form ihrer Nitrate vorgenommen. Dabei wird vorteilhaft das beim Nitrieren erhaltene Gemisch in Eiswasser eingegossen, wobei die 9- und die 11-(Nitro)-apovincaminsäuren in Form ihrer Nitrate ausfallen. Die Gesamtausbeute am Produkt ist mit 75 bis 85% hoch. Die beiden Isomere können am einfachsten durch Kristallisieren ihrer Nitrate getrennt werden. Beim Umkristallisieren des Gemisches aus 50 vol.-%-igem wäßrigem Alkohol fällt zuerst das 9-(Nitro)-apovincaminsäurenitrat aus. Beim Stehenlassen der Mutterlauge fällt zuerst eine geringe Menge Isomergemisch und dann beim Einengen das reine 11-(Nitro)-apovincaminsäurenitrat aus. Aus den auf diese Weise voneinander getrennten Nitraten kann dann die 9- beziehungsweise 11-(Nitro)-apovincaminsäure durch Lösen in einer wäßrigen Lauge und Ausfällen mit der berechneten Menge Säure freigesetzt werden. Bevorzugt werden die Nitrate in mit wäßriger 50%-iger Natronlauge alkalisch gemachtem Äthanol gelöst und die entsprechenden Apovincaminsäurederivate durch Zusatz der berechneten Menge Salzsäure gefällt.

Wie bereits gesagt werden erfindungsgemäß die 9- und/oder 11-(Amino)-apovincaminsäure(n) der Formel(n) Ic und/oder Id aus der beziehungsweise den entsprechenden 9- und/oder 11-(Nitro)-apovincaminsäure(n) der Formel(n) Ia und/oder Ib durch Reduktion hergestellt.

Es wurde nun im Gegensatz zu den Reduktionen des Standes der Technik überraschenderweise festgestellt, daß sich die 9- und/oder 11-(Nitro)-apovincaminsäure(n) der Formel(n) Ia und/oder Ib durch katalytisches Hydrieren quantitativ zu den 9- und/oder 11-(Amino)-apovincaminsäure(n) der

Formel(n) Ic und/oder Id reduzieren lassen. Nach einer vorteilhaften Ausführungsform des erfindungsgemäßen Verfahrens wird daher die Reduktion als katalytisches Hydrieren durchgeführt. Das katalytische Hydrieren kann sowohl in alkalischem als auch in saurem Medium vorgenommen werden, zweckmäßig wird in wäßrig-alkoholischem Medium in der Nähe des Neutralpunktes gearbeitet. Unter diesen Bedingungen entstehen nicht einmal Spuren des in der 14,15-Stellung gesättigten Produktes. Als Katalysator wird bevorzugt Palladium auf Aktivkohle oder Raney-Nickel verwendet. Nach dem Ende des katalytischen Hydrierens wird der Katalysator abfiltriert und das Produkt in an sich bekannter Weise, zum Beispiel durch Eindampfen, isoliert.

Obwohl zur Herstellung der 9- und/oder 11-(Amino)-apovincaminsäure(n) das katalytische Hydrieren wegen seiner Einfachheint und guten Ausbeute am vorteilhaftesten ist, kann die Reduktion auch mit einem chemischen Reduktionsmittel vorgenommen werden [Bruckner Gy.: Szerves Kémia, II/1, S. 469, Tankönyvkiadó Budapest 1977]. Nach einer anderen vorteilhaften Ausführungsform des erfindungsgemäßen Verfahrens wird also die Reduktion mit einem chemischen Reduktionsmittel durchgeführt. Von den in der genannten Schrifttumsstelle aufgeführten Reduktionsmitteln sind diejenigen, welche die Doppelbindung in der 14,15-Stellung nicht sättigen, geeignet. So ist zum Beispiel die Béchamp-Reduktion geeignet, es kann jedoch vorteilhaft auch in Eisessig mit Zink oder in Salzsäure mit Zinn oder Zink reduziert werden, wobei das Reduzieren mit Zinn in salzsaurem Medium bevorzugt ist. Auch die in neutralem Medium durchführbaren Varianten dieser Reduktionen sind geeignet und schließlich kann die Reduktion auch in alkalischem Medium, zum Beispiel mit Natriumdithionit oder Natriumsulfid, vorgenommen werden.

Beim erfindungsgemäßen Verfahren ist daher auch der Reduktionsschritt leicht durchführbar und führt in praktisch quantitativer Ausbeute zu der beziehungsweise den 9- und/oder 11-(Amino)-apovincaminsäure(n) der Formel(n) Ic und/oder Id.

Das Überführen der erfindungsgemäßen 9- beziehungsweise 11-substituierten Apovincaminsäurederivate der allgemeinen Formel I in Säureadditionssalze kann mit Säuren durchgeführt werden. Zu dieser Salzbildung können zum Beispiel die folgenden Säuren verwendet werden: Anorganische Säuren, wie Halogenwasserstoffsäuren, beispielsweise Salzsäure beziehungsweise Bromwasserstoffsäure, Schwefelsäure, Phosphorsäure, Salpetersäure und Überhalogensäuren, beispielsweise Überchlorsäure, sowie organische Säuren, wie Ameisensäure, Essigsäure, Propionsäure, Glykolsäure, Maleinsäure, Hydroxymaleinsäure, Fumarsäure, Bernsteinsäure, Weinsäure, Ascorbinsäure, Citronensäure, Apfelsäure, Salicylsäure, Milchsäure, Zimtsäure, Benzoesäure, Phenylessigsäure, p-Aminobenzoesäure, p-Hydroxybenzoesäure, p-Aminosalicylsäure, Alkylsulfonsäuren, beispielsweise Methansulfonsäure beziehungsweise Äthansulfonsäure, cycloaliphatische Sulfonsäuren, beispielsweise Cyclohexylsulfonsäure, Arylsulfonsäuren, beispielsweise p-Toluolsulfonsäure, Naphthalinsulfonsäure beziehungsweise Sulfanilsäure, und schließlich Aminosäuren, beispielsweise Asparaginsäure, Glutaminsäure, N-Acetylasparaginsäure beziehungsweise N-Acetylglutarsäure.

Die Salzbildung kann in einem inerten, vorzugsweise organischen, Lösungsmittel, zum Beispiel einem aliphatischen Alkohol mit 1 bis 6 Kohlenstoffatom(en), vorgenommen werden. Dazu können die racemischen oder optisch aktiven 9- beziehungsweise 11-substituierten Apovincaminsäurederivate der allgemeinen Formel I in dem Lösungsmittel gelöst und die entsprechende Säure oder deren Lösung im selben Lösungsmittel zugesetzt werden, wobei das Gemisch schwach sauer wird (pH-Wert etwa 5 bis 6). Dann wird das ausgefallene Säureadditionssalz in geeigneter Weise, zum Beispiel durch Filtrieren, abgetrennt.

Die erfindungsgemäßen 9- beziehungsweise 11-substituierten Apovincaminsäurederivate der allgemeinen Formel I bilden infolge ihres Säurecharakters auch mit anorganischen Basen Salze. Die Herstellung dieser Metallsalze, vorzugsweise Alkali- beziehungsweise Erdalkalimetallsalze, wie Natrium-, Kalium- beziehungsweise Calciumsalze, beziehungsweise Ammoniumsalze, erfolgt nach den bekannten Verfahrensweisen der Salzbildung. Als anorganische Basen werden bevorzugt Alkalimetall- beziehungsweise Erdalkalimetallhydroxyde, zum Beispiel Natrium-, Kalium- oder Calciumhydroxyd, beziehungsweise Ammoniumhydroxyd verwendet.

Aus den 9- beziehungsweise 11-substituierten Apovincaminsäurederivaten der allgemeinen Formel I können die monoquaternären Salze zweckmäßig durch Umsetzen mit Alkylhalogeniden, vorzugsweise Alkylbromiden oder Alkyljodiden, in äquivalenter Menge oder leichtem Überschuß hergestellt werden. Die Bildung der quaternären Salze wird vorteilhaft in einem inerten organischen Lösungsmittel unter Erwärmen durchgeführt.

Die erfindungsgemäßen racemischen beziehungsweise optisch aktiven Apovincaminsäurederivate der allgemeinen Formel I beziehungsweise ihre Salze können gegebenfalls weiter gereinigt, zum Beispiel umkristallisiert, werden. Das zum Umkristallisieren verwendete Lösungsmittel wird unter Berücksichtigung der Lösungs- und Kristallisationseigenschaften der jeweiligen Substanz gewählt.

Die im erfindungsgemäßen Verfahren als Ausgangssubstanz verwendete Apovincaminsäure ist eine bekannte Verbindung, die zum ersten Mal in der ungarischen Patentschrift 160 367 beschrieben wurde.

Die erfindungsgemäßen 9- beziehungsweise 11-substituierten Apovincaminsäurederivate der allgemeinen Formel I beziehungsweise ihre Salze sind, außer selbst therapeutisch wirksam zu sein, wertvolle Zwischenprodukte zur Herstellung von im Ring A substituierten biologisch aktiven Eburnanderivaten, insbesondere zur Herstellung der in den EP 170 926 und EP 174 459 beschriebenen Nitroapovincaminsäureester beziehungsweise eine, gegebenenfalls substituierte, Aminogruppe aufweisenden Aminoapovincaminsäurederivate.

Die Eigenschaften der erstgenannten neuen erfindungsgemäßen Verbindungen sind die Ursache für die überlegenen pharmakologischen Wirkungen der aus ihnen herstellbaren neuen Nitroapovincaminsäureester beziehungsweise eine, gegebenenfalls substituierte, Aminogruppe, aufweisenden Aminoapovincaminsäurederivate.

Die Weiterverarbeitung zu diesen beziehungsweise zu den bekannten 9- beziehungsweise 11-(Nitro)-apovincamin und 9- beziehungsweise 11-(Amino)-apovincamin kann wie folgt durchgeführt werden.

1. Weiterverarbeitung zu 9- beziehungsweise 11-(Nitro)-apovincaminsäurederivaten der allgemeinen Formel

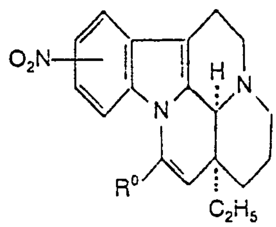

III,

worin

$R^0$ für einen Halogencarbonylrest der allgemeinen Formel

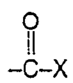

IV

in welch letzterer

X ein Halogenatom bedeutet, oder einen Kohlenwasserstoffcarbonylrest der allgemeinen Formel

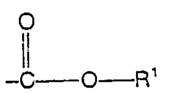

V

in welch letzterer

$R^1$ einen, gegebenenfalls ein- oder mehrfach substituierten, aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet, oder einen Aminocarbonylrest der allgemeinen Formel

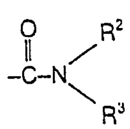

VI

in welch letzterer

$R^2$ und $R^3$ unabhängig voneinander Wasserstoff beziehungsweise Alkylreste mit 1 bis 8 Kohlenstoffatom(en), welche gegebenenfalls 2 miteinander verbunden als Alkylenrest zusammen mit dem Stickstoffatom, an welches sie gebunden sind, einen, gegebenenfalls durch 1 oder mehr weitere[s] Stickstoffatom(e) oder sonstige[s] Heteroatom(e) aufweisenden gesättigten heterocyclischen Rest darstellen, bedeuten oder

$R^2$ Wasserstoff bedeutet und

$R^3$ eine Aminogruppe darstellt,

oder

eine Cyanogruppe steht

und die Nitrogruppe in der 9- oder 11-Stellung ist, sowie von ihren optisch aktiven Isomeren und Salzen. Umsetzen der racemischen oder optisch aktiven 9- beziehungsweise 11-(Nitro)-apovincaminsäuren der allgemeinen Formel I mit R = Nitrogruppe in der 9- oder 11-Stellung beziehungsweise Salzen derselben mit anorganischen Basen der allgemeinen Formel

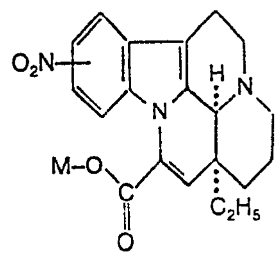

I′

worin M für Wasserstoff, ein Metallatom oder eine Ammoniumgruppe steht, oder, falls M von einem Metallatom verschieden ist, auch deren Säureadditionssalzen beziehungsweise quaternären Salzen mit Hydroxyverbindungen beziehungsweise Estern der allgemeinen Formel

$$R^1\text{–}Y \qquad \text{VII}$$

worin

$R^1$ die oben angegebenen Bedeutungen hat

und

Y für eine Hydroxygruppe oder einen Säurerest steht,

gegebenenfalls in Gegenwart von Basen, oder mit Halogenierungsmitteln und gegebenenfalls erfolgendes Umsetzen der erhaltenen 9- beziehungsweise 11-(Nitro)-apovincaminsäurederivate der allgemeinen Formel III, bei welche $R^0$ für einen Halogencarbonylrest der allgemeinen Formel IV steht, mit nukleophilen Reagenzien der allgemeinen Formel

$$H\text{–}O\text{–}R^1 \qquad \text{VIII}$$

beziehungsweise

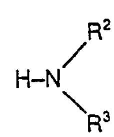

IX

worin $R^1$, $R^2$ und $R^3$ die oben angegebenen Bedeutungen haben, und gegebenenfalls erfolgende Dehydratisieren der erhaltenen 9- beziehungsweise 11-(Nitro)-apovincaminsäurederivate der allgemeinen Formel III, bei welche $R^0$ für einen Aminocarbonylrest der allgemeinen Formel VI, bei welchem $R^2$ und $R^3$ Wasserstoffatome bedeuten, steht, mit wasserentziehenden Mitteln sowie in an sich bekannter Weise gegebenenfalls erfolgendes Umestern oder Amidieren der erhaltenen 9- beziehungsweise 11-(Nitro)-apovincaminsäurederivate der allgemeinen Formel III, bei welchen $R^0$ für einen Kohlenwasserstoffoxycarbonylrest der allgemeinen Formel V, bei welchem $R^1$ einen Alkylrest mit 1 oder 2 Kohlenstoffatom(en) bedeutet, steht, beziehungsweise ihrer Säureadditionssalze und/oder in an sich bekannter Weise gegebenenfalls erfolgendes Überführen der erhaltenen 9- beziehungsweise 11-(Nitro)-apovincaminsäurederivate der allgemeinen Formel III in Säureadditionssalze oder quaternäre Salze und/oder in an sich bekannter Weise gegebenenfalls erfolgendes Überführen der erhaltenen Salze der 9- beziehungsweise 11-(Nitro)-apovincaminsäurederivate der allgemeinen Formel III in die freien 9- beziehungsweise 11-(Nitro)-apovincaminsäurederivate der allgemeinen Formel III oder in andere Säureadditionssalze beziehungsweise quaternäre Salze und/oder gegebenenfalls erfolgende Vornahme einer Spaltung der erhaltenen racemischen 9- beziehungsweise 11-(Nitro)-apovincaminsäurederivate der allgemeinen Formel III beziehungsweise von deren Säureadditionssalzen beziehungsweise quaternären Salzen beziehungsweise einer Racemisierung der entsprechenden optisch aktiven Verbindungen in Stereoisomere. Dieses Verfahren ist in der EP 174 459 näher beschrieben. Durch die erfindungsgemäßen neuen Zwischenprodukte 9- beziehungsweise 11-(Nitro)-apovincaminsäuren beziehungsweise Salze derselben wird also ein neuer Weg durch ein chemisch eigenartiges Verfahren zur Herstellung der therapeutisch wertvollen Endprodukte 9- beziehungsweise 11-(Nitro)-apovincaminsäurederivate der allgemeinen Formel III beziehungsweise ihre Salze, der technisch fortschrittlich ist, eröffnet.

2. Weiterverarbeitung zu in der 9- oder 11-Stellung eine, gegebenenfalls substituierte, Aminogruppe aufweisenden Apovincaminsäurederivaten der allgemeinen Formel

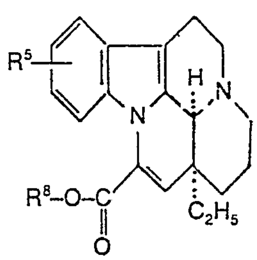

X

worin

$R^5$ für eine Aminogruppe

oder

einen Acylamidorest der allgemeinen Formel

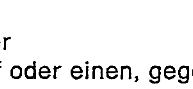

XI

in welch letzterer

$R^6$ Wasserstoff oder einen, gegebenenfalls ein- oder mehrfach substituierten, aliphatischen Kohlenwasserstoffrest mit 1 bis 10 Kohlenstoffatom(en), cycloaliphatischen Kohlenwasserstoffrest mit 3 bis 8 Kohlenstoffatomen, aromatischen Kohlenwasserstoffrest mit 6 bis 14 Kohlenstoffatomen oder 1 oder mehr gleiche oder verschiedene Heteroatom(e) aufweisenden heteroaromatischen Kohlenwasserstoffrest bedeutet,

oder einen Sulfonylamidorest der allgemeinen Formel

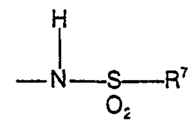

XII

in welch letzterer

$R^7$ einen, gegebenenfalls ein- oder mehrfach substituierten, aliphatischen Kohlenwasserstoffrest mit 1 bis 10 Kohlenstoffatom(en) oder aromatischen Kohlenwasserstoffrest mit 6 bis 14 Kohlenstoffatomen bedeutet,

steht,

$R^8$ Wasserstoff oder einen, gegebenenfalls ein- oder mehrfach substituierten, aliphatischen Kohlenwasserstoffrest mit 1 bis 10 Kohlenstoffatom(en), cycloaliphatischen Kohlenwasserstoffrest mit 3 bis 8 Kohlenstoffatomen oder aromatischen Kohlenwasserstoffrest mit 6 bis 14 Kohlenstoffatomen bedeutet,

und

$R^5$ in der 9- oder 11-Stellung ist, mit der weiteren Maßgabe, daß im Falle daß

$R^5$ für eine Aminogruppe steht,

$R^8$ von einem Methylrest verschieden ist,

sowie ihrer optisch aktiven Isomere und Salze.

a) In an sich bekannter Weise erfolgendes Acylieren der racemischen oder optisch aktiven 9- beziehungsweise 11-(Amino-apovincaminsäure der Formel

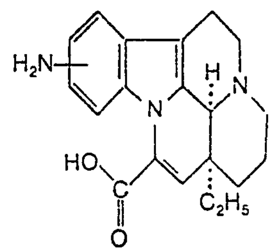

I''

(Formel I mit R = Aminogruppe),

worin die Aminogruppe in der 9- oder 11-Stellung ist, oder von deren Salzen und gegebenenfalls erfolgendes Verestern oder Umestern der enthaltenen in der 9- oder 11-Stellung eine, gegebenenfalls substituierte, Aminogruppe aufweisenden Apovincaminsäurederivate der allgemeinen Formel X, bei welchen $R^5$ für einen Acylamidorest der allgemeinen Formel XI oder einen Sulfonylamidorest der allgemeinen Formel XII, wobei $R^6$ und $R^7$ die oben angegebenen Bedeutungen haben, steht,

oder

b) in an sich bekannter Weise erfolgendes Verestern beziehungsweise Umestern der racemischen oder optisch aktiven in der 9- oder 11-Stellung eine, gegebenenfalls substituierte, Aminogruppe aufweisenden Apovincaminsäurederivate der Formel

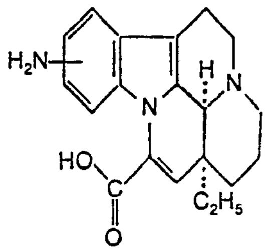

I″

(Formel I mit R = Aminogruppe), worin die Aminogruppe in der 9- oder 11-Stellung ist, oder von deren Salzen und gegebenenfalls erfolgendes Acylieren der erhaltenen in der 9- oder 11-Stellung eine, gegebenenfalls substituierte, Aminogruppe aufweisenden Apovincaminsäurederivate der allgemeinen Formel X, bei welchen $R^5$ für eine Aminogruppe steht und $R^8$ die oben angegebenen Bedeutungen haben, beziehungsweise von ihren Salzen oder

c) Reduzieren von nach dem Abschnitt 1) erhaltenen racemischen oder optisch aktiven 9- oder 11-(Nitro)-apovincaminsäurederivaten der allgemeinen Formel

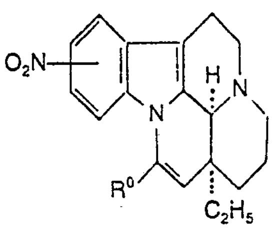

III,

worin $R^0$ die oben angegebenen Bedeutungen, ausgenommen einen Kohlenwasserstoffoxycarbonylrest mit $R^1$ = nicht substituierter Methylrest, hat und die Nitrogruppe in der 9- oder 11-Stellung ist, und gegebenenfalls erfolgende Durchführung der fakultativen Umsetzung(en) des beziehungsweise der obigen Abschnitte[s] a) und/oder b) sowie in an sich bekannter Weise gegebenenfalls erfolgendes Überführen der erhaltenen in der 9- oder 11-Stellung eine, gegebenenfalls substituierte, Aminogruppe aufweisenden Apovincamisäurederivate der allgemeinen Formel X in Säureadditionssalze oder quaternäre Salze und/oder in an sich bekannter Weise gegebenenfalls erfolgendes Überführen der erhaltenen Salze der in der 9- oder 11-Stellung eine, gegebenenfalls substituierte, Aminogruppe aufweisenden Apovincaminsäurederivate der allgemeinen Formel X in die freien in der 9- oder 11-Stellung eine, gegebenenfalls substituierte, Aminogruppe aufweisenden Apovincaminsäurederivate der allgemeinen Formel X oder in andere Säureadditionssalze beziehungsweise quaternäre Salze und/oder gegebenenfalls erfolgende Vornahme einer Spaltung der erhaltenen racemischen in der 9- oder 11-Stellung eine, gegebenenfalls substituierte, Aminogruppe aufweisenden Apovincaminsäurederivate der allgemeinen Formel X beziehungsweise von deren Säureadditionssalzen beziehungsweise quaternären Salzen beziehungsweise eine Racemisierung der entsprechenden optisch aktiven Verbindungen in Stereoisomere. Dieses Verfahren ist in den EP 170 926 und EP 174 459 näher beschrieben.

Durch die erfindungsgemäßen neuen Zwischenprodukte 9- beziehungsweise 11-(Nitro)-apovincaminsäuren beziehungsweise Salze derselben wird somit ein neuer Weg durch ein chemisch eigenartiges Verfahren zur Herstellung der therapeutisch wertvollen Endprodukte in der 9- oder 11-Stellung eine, gegebenenfalls substituierte, Aminogruppe aufweisenden Apovincaminsäurederivate der allgemeinen Formel X beziehungsweise ihrer Salze, der technisch fortschrittlich ist, eröffnet.

Es ist auch besonders vorteilhaft, daß die erfindungsgemäßen 9- beziehungsweise 11-(Amino)-apovincaminsäuren der allgemeinen Formel I″, das heißt der allgemeinen Formel I mit R = Aminogruppe, beziehungsweise ihre Salze, insbesondere die durch katalytisches Hydrieren hergestellten, ohne zwischenzeitliches Isolieren zu biologisch aktiven Eburnanverbindungen, zum Beispiel zu den entsprechenden in der 9- oder 11-Stellung eine, gegebenenfalls substituierte, Aminogruppe aufweisenden Aminoapovincaminsäurederivaten der allgemeinen Formel X umgesetzt werden können. Diese Möglichkeit ist in erster Linie dadurch gegeben, daß das erfindungsgemäße Verfahren sehr reine Produkte in hohen Ausbeuten liefert.

Die Erfindung wird an Hand der folgenden Beispiele näher erläutert.

Beispiel 1

(+)-9-Nitroapovincaminsäure

Die Lösung von 32,2 g (0,1 Mol) (+)-Apovincaminsäure ($[\alpha]_D^{20}$ = +220,48, c = 2, Pyridin) in 180 ml Eisessig wird auf 16 °C gekühlt und unter Rühren innerhalb von 5–15 Minuten mit dem Gemisch von 52 ml Eisessig und 52 ml rauchender Salpetersäure (spez. Gewicht: 1,52) versetzt, wobei darauf zu achten ist, daß die Temperatur des Gemisches nicht über 16 °C ansteigt. Bei der gleichen Temperatur wird das Gemisch noch 50 Minuten lang gerührt und dann in 1 Liter Eiswasser gegossen. Die ausgeschiedenen Kristalle werden abfiltriert und auf dem Filter dreimal mit je 100 ml Einswasser und dann mit 50 ml Äther gewaschen. Als Rohprodukt wird das Nitrat der Titelverbindung erhalten (32,2 g = 75%). Das rohe Nitrat wird in 800 ml 50 vol.-%-igem Äthanol warm gelöst, die Lösung mit Aktivkohle geklärt und dann über Nacht stehengelassen. Die ausgefallenen Kristalle werden abfiltriert und dreimal mit je 20 ml 50 vol.-%-igem Äthanol gewaschen. Man erhält 12,8 g (29%) (+)-Nitroapovincaminsäurenitrat, das bei 232–234 °C schmilzt.

Die kristalline Substanz wird bei 60–70 °C in 160 ml 50 vol.-%-igem Äthanol gelöst, und während des Lösungsprozesses wird der pH-Wert der Lösung mit 1 n Natriumhydroxydlösung auf 7,5 eingestellt. Dann wird die Lösung bis zum Erreichen eines pH-Wertes von 6,5 mit 10%iger Salzsäure versetzt. Die Lösung wird gekühlt, wobei sich gelbe Kristalle abscheiden. Diese werden abfiltriert und dreimal mit je 20 ml Wasser gewaschen. Man erhält 7,4 g (20%) (+)-9-Nitroapovincaminsäure. Nach Umkristallisieren aus einem im Volumverhältnis 1:1 bereiteten Gemisch aus Pyridin und Äthanol schmilzt das Produkt bei 260–262 °C.

$[\alpha]_D$ = +317,32 (c = 0,4; Pyridin).

UV-Spektrum (in salzsäurehaltigem Äthanol): λ(nm): 210 (4,46), 287 (4,01); $^1$H NMR-Spektrum (DMSO-$d_6$): δ Et 0,96(t)3, 1,90(q)2, H-3 4,52(s)1, H-15 6,26(s)1, H-10 7,90(dd)1, (J = 8 und 1 Hz), H-11 7,30(t)1 (J = 8 Hz), H-12 7,74(dd)1 (J = 8 und 1 Hz).

Elementaranalyse für $C_{20}H_{21}N_3O_4$ (M = 367,39)
berechnet, %: C 65,38 H 5,76 N 11,43
gefunden, %: C 65,30 H 6,00 N 11,44.

Aus der nach der Abtrennung des reinen (+)-9-Nitroapovincaminsäurenitrates zurückbleibenden Mutterlauge wird auf die im Beispiel 2 beschriebene Weise (+)-11-Nitroapovincaminsäure hergestellt.

Beispiel 2

(+)-11-Nitroapovincaminsäure

Die Lösung von 32,2 g (0,1 Mol) (+)-Apovincaminsäure in 180 ml Eisessig wird mit 77 ml Chloroform versetzt und dann auf 0 °C gekühlt. Unter Rühren gibt man zu der Lösung das Gemisch von 52 ml Eisessig und 52 ml rauchender Salpetersäure (spez. Gewicht 1,52), wobei darauf zu achten ist, daß die Temperatur des Gemisches auf 0 °C bleibt. Bei dieser Temperatur wird das Gemisch noch eine Stunde lang gerührt und dann in 1 Liter Eiswasser gegossen. Von der erhaltenen dicken, klebrigen Masse wird das Wasser dekantiert, und der Rückstand wird mit 200–300 ml Eiswasser verrieben. Das Wasser wird verworfen. Der Kristallbrei wird mit 200–300 ml Äther verrieben, abfiltriert, auf dem Filter dreimal mit je 100 ml Eiswasser und dann mit 50 ml Äther gewaschen und schließlich getrocknet. Man erhält 37,8 g (85%) rohes 9-Nitroapovincaminsäurenitrat. Die Substanz wird warm in 900 ml 50-%igem wäßrigem Äthanol gelöst, die Lösung geklärt und dann über Nacht bei Raumtemperatur stehengelassen. Die ausgefallenen Kristalle werden abfiltriert, dreimal mit je 20 ml 50 vol.-%-igem Äthanol gewaschen und dann getrocknet. Man erhält 7,9 g 9-Nitroapovincaminsäurenitrat. Aus der Mutterlauge scheidet sich bis zum nächsten Tag weiteres kristallines 9-Nitroapovincaminsäurenitrat ab. Dieses wird abfiltriert, dreimal mit je 10 ml 50 vol.-%-igem wäßrigem Äthanol gewaschen und dann getrocknet. Gewicht: 1 g.

Die Mutterlauge und die vereinigten Waschflüssigkeiten werden auf die Hälfte ihres Volumens eingeengt, die ausfallenden Kristalle werden abfiltriert und dreimal mit je 50 ml Wasser gewaschen. Man erhält 24,9 g (58%) (+)-11-Nitroapovincaminsäurenitrat, das bei 211–214 °C schmilzt.

Das erhaltene Salz wird bei 60–70 °C unter Zusatz von etwa 60 ml 1 n Natronlauge in 500 ml 50 vol.-%-igem Äthanol gelöst. Falls erforderlich, wird der pH-Wert auf 7 eingestellt. Die Lösung wird gekühlt, wobei sich gelbe Kristalle ausscheiden. Diese werden abfiltriert und dreimal mit je 40 ml Wasser gewaschen. Man erhält 15 g (40%) 11-Nitroapovincaminsäure, die nach Umkristallisieren aus einem im Volumverhältnis 1:1 bereiteten Gemisch von Pyridin und Äthanol bei 250–254 °C schmilzt.

$[\alpha]_D$ = +187,43 (c = 0,4; Pyridin).

UV-Spektrum (in salzsaurem Äthanol): λ(nm): 212 (4,32), 254 (4,13), 316 (3,98). $^1$H–NMR-Spektrum (DMSO–$d_6$) δ: Et 0,93(t)3, 1,9 (überlagert vom Signal des Lösungsmittels), H-3 4,20(s)1; H-15 6,20(s)1, H-9 7,60(d)1 (J = 9 Hz), H-10 7,93(dd)1 (J = 9 und 2 Hz), H-12 8,38(d)1 (J = 2 Hz).

Elementaranalyse für $C_{20}H_{21}N_3O_4$ (M = 367,39)
berechnet, %: C 65,38 H 5,76 N 11,43
gefunden, %: C 65,42 H 5,84 N 11,40.

Beispiel 3

(−)-9-Aminoapovincaminsäure

3,6 g (0,01 Mol) nach der Verfahrensweise des Beispiels 1 hergestellte (+)-9-Nitroapovincaminsäure werden in einem Gemisch aus 10 ml Äthanol und 5 ml Wasser gelöst. Die Lösung wird mit 0,04 g 10%iger Pd-Aktivkohle versetzt und bei Raumtemperatur in einer Wasserstoffatmosphäre gerührt. Nachdem 0,03 Mol Wasserstoff aufgenommen waren, wird der Katalysator abfiltriert und dreimal mit je 2 ml 50%igem wäßrigem Äthanol gewaschen. Filtrat und Waschflüssigkeit werden vereinigt. Die erhaltene Lösung kann gewünschtenfalls unmittelbar zur Herstellung der entsprechenden Acylamidoderivate eingesetzt werden.

Zur Isolierung der (−)-9-Aminoapovincaminsäure wird die Lösung im Vakuum eingedampft. Der Rückstand wird mit 20 ml Äthanol verrieben, die Kristalle werden abfiltriert, zweimal mit je 3 ml Äthanol gewaschen und dann getrocknet. Man erhält 2,85 g (85%) der Titelverbindung, die bis zu 340 °C nicht schmilzt.

$[\alpha]_D$ = −25° (c = 0,4; Pyridin).

UV-Spektrum (in salzsäurehaltigem Äthanol) λ(nm): 210 (4,43, 222 (4,53), 263 (4,05), 333 (3,78). $^1$H–NMR-Spektrum (DMSO–$d_6$) δ: Et 0,93(3), ca. 1,8(2), H-3 4,44(s)1, $NH_2OH$ 4,75(3), H-15 5,68(s)1, H-10 6,25, H-11, H-12 6,72s(1–1), Gerüstprotonen 1,2–3,4.

Massespektrum M: (rel. Intensität, %): 337(67), 308(67), 295(18), 293(55), 267(92), 264(92), 223(100).

Beispiel 4

(+)-11-Aminoapovincaminsäure

Zu der Lösung von 3,6 g (0,01 Mol) (+)-11-Nitroapovincaminsäure in einem Gemisch aus 5 ml Äthanol und 10 ml 1 n wäßriger Natronlauge werden 0,04 g 10%iger Pd-Aktivkohlekatalysator gegeben. Die Lösung wird bei Raumtemperatur in einer Wasserstoffatmosphäre gerührt. Nachdem 0,03 Mol (720 ml) Wasserstoff aufgenommen waren, wird der Katalysator abfiltriert und dreimal mit je 2 ml 50-%igem wäßrigem Äthanol gewaschen. Filtrat und Waschflüssigkeit werden vereinigt. Die Lösung kann gewünschtenfalls unmittelbar zur Herstellung der entsprechenden Acylamidoderivate verwendet werden.

Vor der Isolierung der (+)-11-Aminoapovincaminsäure wird die Lösung mit 10 ml 1 n wäßriger Salzsäure versetzt und im Vakuum auf die Hälfte ihres Volumens eingeengt. Die konzentrierte Lösung wird über Nacht stehengelassen. Das auskristallisierte Produkt wird abfiltriert und mit Eiswasser gewaschen. Man erhält 3,1 g (92%) der Titelverbindung, die bei 200–202 °C unter Zersetzung schmilzt.

$[\alpha]_D$ = +77,89° (c = 0,4; Pyridin).
UV-Spektrum (in salzsäurehaltigem Äthanol): λ(nm): 210 (4,32), 220 (4,43), 272 (3,98), 313 (3,77).
$^1$H-NMR-Spektrum (DMSO–$d_6$) δ: Et 0,90t(3), etwa 1,80q(2), H-3 4,29s(1), H-15 5,72s(1), H-9 7,10d(1) (J = 9 Hz), H-10 6,43dd(1), H-12 6,64d(1) (J = 2 Hz).

Massespektrum M ($C_{20}H_{23}N_3O_2$ rel. Int.%): 337(54), 308(85), 292(15), 267(100).

Beispiel 5
(+)-11-Aminoapovincaminsäure

7,3 g (0,02 Mol) (+)-11-Nitroapovincaminsäure werden in einem Gemisch aus 100 ml konzentrierter Salzsäure und 50 ml Wasser gelöst. Zu der Lösung gibt man 7,3 g Zinnstaub und läßt eine Viertelstunde kochen. Die Lösung wird vom verbliebenen Zinn dekantiert und mit 50 ml Wasser verdünnt. Dann wird so lange Schwefelwasserstoff in die Lösung geleitet, bis sich ein dunkelbrauner Niederschlag abscheidet. Das Gemisch wird aufgekocht, dann auf 20 °C abgekühlt und durch Filtrieren vom Zinnsulfid befreit. Die klare Lösung wird im Vakuum auf ein Drittel ihres Volumens eingedampft. Die konzentrierte Lösung wird über Nacht im Kühlschrank stehengelassen. Das auskristallisierte Produkt wird abfiltriert und mit Eiswasser gewaschen. Man erhält 5,8 g (86%) der Titelverbindung, die bei Vermischen mit dem Produkt gemäß Beispiel 4 keine Schmelzpunktdepression verursacht.

Beispiel 6
(+)-11-Nitroapovincaminsäure

3,6 g (0,01 Mol) (+)-11-Nitroapovincaminsäure werden in 200 ml Äthanol gelöst, das 0,56 g Kaliumhydroxyd enthält. Die Lösung wird eingedampft und der Rückstand aus einem Gemisch von 100 ml Aceton und 5 ml Äthanol umkristallisiert. 3,2 g des im Titel genannten Kaliumsalzes werden in Form einer gelben kristallinen Substanz erhalten. Schmelzpunkt: schmilzt bis 340 °C nicht.

Beispiel 7
(+)-11-Nitroapovincaminsäure-oxalat

Eine Lösung von 0,36 g (0,001 Mol) (+)-11-Nitroapovincaminsäure in 30 ml heißem Wasser wird mit konzentriertem Ammoniak auf einen pH-Wert von 7,8 eingestellt. Die heiße Lösung wird mit einer konzentrierten wäßrigen Lösung von Oxalsäure auf pH 3 angesäuert und dann auf Raumtemperatur abgekühlt. Die ausgefallenen Kristalle werden abfiltriert und mit kaltem Wasser gewaschen. Man erhält 0,35 g (78%) des Oxalats. Schmelzpunkt: 278–280 °C.

**Patentansprüche für die Vertragsstaaten BE, CH, DE, FR, GB, IT, LI, NL, SE**

1. Racemische oder optisch aktive 9- beziehungsweise 11-substituierte Apovincaminsäurederivate der allgemeinen Formel

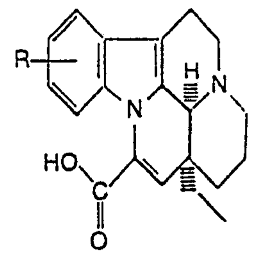

I

worin
R für eine Nitro- oder Aminogruppe in der 9- oder 11-Stellung steht,
sowie ihre Salze.

2. Die 9- beziehungsweise 11-substituierten Apovincaminsäurederivate
(+)-9-(Nitro)-apovincaminsäure,
(+)-11-(Nitro)-apovincaminsäure,
(−)-9-(Amino)-apovincaminsäure und
(+)-11-Aminoapovincaminsäure
sowie ihre Salze.

3. Verfahren zur Herstellung der 9- beziehungsweise 11-substituierten Apovincaminsäurederivate nach Anspruch 1 oder 2, dadurch gekennzeichnet, daß man Apovincaminsäure der Formel

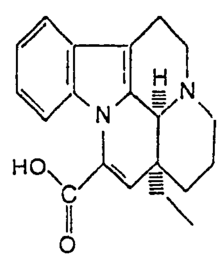

II

nitriert und gegebenenfalls die erhaltene(n) 9- und/oder 11-(Nitro)-apovincaminsäure(n), gegebenenfalls nach ihrem Isolieren aus dem Reaktionsgemisch, reduziert sowie gegebenenfalls die erhaltenen 9- beziehungsweise 11-substituierten Apovincaminsäurederivate der allgemeinen Formel I in Säureadditionssalze oder quaternäre Salze beziehungsweise Metall- beziehungsweise Ammoniumsalze überführt und/oder die erhaltenen Salze der 9- beziehungsweise 11-substituierten Apovincaminsäurederivate der allgemeinen Formel I in die freien 9- beziehungsweise 11-substituierten Apovincaminsäurederivate der allgemeinen Formel I oder in andere Säureadditionssalze beziehungsweise quaternäre Salze beziehungsweise Metall- beziehungsweise Ammoniumsalze überführt und/oder gegebenenfalls eine Spaltung der erhaltenen racemischen 9- beziehungsweise 11-substituierten Apovincaminsäurederivate der allgemeinen Formel I beziehungsweise von deren Säureadditionssalzen beziehungsweise quaternären Salzen beziehungsweise Metall- beziehungsweise Ammoniumsalzen in ihre optischen Antipoden beziehungsweise eine

Racemisierung der entsprechenden optisch aktiven Verbindungen in Stereoisomere vornimmt.

4. Verfahren nach Anspruch 3, dadurch gekennzeichnet, daß man das Nitrieren mit konzentrierter Salpetersäure in eisessigsaurem Medium durchführt.

5. Verfahren nach Anspruch 3 oder 4, dadurch gekennzeichnet, daß man das Nitrieren in 1 bis 3 Vol.-% Acetonitril oder Dimethylformamid enthaltendem Eisessig durchführt.

6. Verfahren nach Anspruch 3 oder 4, dadurch gekennzeichnet, daß man das Nitrieren in 10 bis 50 Vol.-% Chloroform enthaltendem Eisessig durchführt.

7. Verfahren nach Anspruch 3 bis 6, dadurch gekennzeichnet, daß man das Nitrieren bei Temperaturen von 0 bis +16 °C durchführt.

8. Verfahren nach Anspruch 3 bis 7, dadurch gekennzeichnet, daß man das Isolieren der 9- und/oder 11-(Nitro)-apovincaminsäure(n) in Form ihrer Nitrate vornimmt.

9. Verfahren nach Anspruch 3 bis 8, dadurch gekennzeichnet, daß man die Reduktion als katalytisches Hydrieren, insbesondere in Gegenwart von Palladium auf Aktivkohle oder Raney-Nickel, vor allem bei annähernd neutralem pH-Wert, durchführt.

10. Verfahren nach Anspruch 3 bis 8, dadurch gekennzeichnet, daß man die Reduktion mit einem chemischen Reduktionsmittel, insbesondere Zinn in salzsaurem Medium, durchführt.

**Patentansprüche für den Vertragsstaat: AT**

1. Verfahren zur Herstellung von racemischen oder optisch aktiven 9- beziehungsweise 11-substituierte Apovincamisäurederivaten der allgemeinen Formel

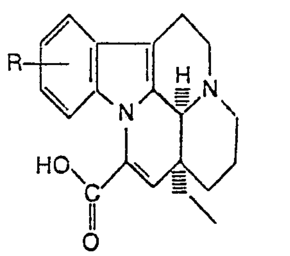
I

worin

R für eine Nitro- oder Aminogruppe in der 9- oder 11-Stellung steht,

sowie ihrer Salze, dadurch gekennzeichnet, daß man Apovincaminsäure der Formel

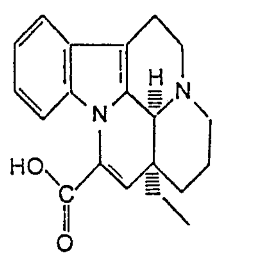
II

nitriert und gegebenenfalls die erhaltene(n) 9- und/oder 11-(Nitro)-apovincaminsäure(n), gegebenenfalls nach ihrem Isolieren aus dem Reaktionsgemisch, reduziert sowie gegebenenfalls die erhaltenen 9- beziehungsweise 11-substituierten Apovincaminsäurederivate der allgemeinen Formel I in Säureadditionssalze oder quaternäre Salze beziehungsweise Metall- beziehungsweise Ammoniumsalze überführt und/oder die erhaltenen Salze der 9- beziehungsweise 11-substituierten Apovincaminsäurederivate der allgemeinen Formel I in die freien 9- beziehungsweise 11-substituierten Apovincaminsäurederivate der allgemeinen Formel I oder in andere Säureadditionssalze beziehungsweise quaternäre Salze beziehungsweise Metall- beziehungsweise Ammoniumsalze überführt und/oder gegebenenfalls eine Spaltung der erhaltenen racemischen 9- beziehungsweise 11-substituierten Apovincaminsäurederivate der allgemeinen Formel I beziehungsweise von deren Säureadditionssalzen beziehungsweise quaternären Salzen beziehungsweise Metall- beziehungsweise Ammoniumsalzen in ihre optischen Antipoden beziehungsweise eine Racemisierung der entsprechenden optisch aktiven Verbindungen in Stereoisomere vornimmt.

2. Verfahren nach Anspruch 1, dadurch gekennzeichnet, daß man das Nitrieren mit konzentrierter Salpetersäure in eisessigsaurem Medium durchführt.

3. Verfahren nach Anspruch 1 oder 2, dadurch gekennzeichnet, daß man das Nitrieren in 1 bis 3 Vol.-% Acetonitril oder Dimethylformamid enthaltendem Eisessig durchführt.

4. Verfahren nach Anspruch 1 oder 2, dadurch gekennzeichnet, daß man das Nitrieren in 10 bis 50 Vol.-% Chloroform enthaltendem Eisessig durchführt.

5. Verfahren nach Anspruch 1 bis 4, dadurch gekennzeichnet, daß man das Nitrieren bei Temperaturen von 0 bis +16 °C durchführt.

6. Verfahren nach Anspruch 1 bis 5, dadurch gekennzeichnet, daß man das Isolieren der 9- und/oder 11-(Nitro)-apovincaminsäure(n) in Form ihrer Nitrate vornimmt.

7. Verfahren nach Anspruch 1 bis 6, dadurch gekennzeichnet, daß man die Reduktion als katalytisches Hydrieren, insbesondere in Gegenwart von Palladium auf Aktivkohle oder Raney-Nickel, vor allem bei annähernd neutralem pH-Wert, durchführt.

8. Verfahren nach Anspruch 1 bis 6, dadurch gekennzeichnet, daß man die Reduktion mit einem chemischen Reduktionsmittel, insbesondere Zinn in salzsaurem Medium, durchführt.

9. Verwendung der gemäß den Ansprüchen 1 bis 8 hergestellten racemischen oder optisch aktiven 9- beziehungsweise 11-substituierten Apovincaminsäurederivaten der allgemeinen Formel (I) zur Herstellung von Arzneimitteln.

**Claims for the Contracting States: BE, CH, DE, FR, GB, IT, LI, NL, SE**

1. Racemic or optically active 9- and/or 11-substituted apovincamic acid derivatives having the general formula

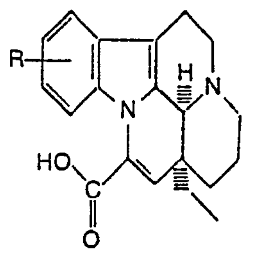

I

wherein

R is a nitro or amino group in position 9 or 11, and the salts thereof.

2. The 9- and/or 11-substituted apovincamic acid derivatives (+)-9-(nitro)-apovincamic acid, (+)-11-(nitro)-apovincamic acid, (−)-9-(amino)-apovincamic acid and (+)-11-aminoapovincamic acid and the salts thereof.

3. A process of producing the 9- and/or 11-substituted apovincamic acid derivatives according to claim 1 or 2, characterized in that apovincamic acid having the formula

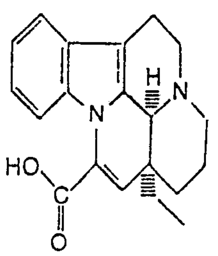

II

is nitrated and the resulting 9- and/or 11-(nitro)-apovincamic acid(s) is or are optionally reduced, optionally after it has or they have been isolated from the reaction mixture and, optionally
the resulting 9- and/or 11-substituted apovincamic acid derivatives having the general formula I are transformed to acid addition salts or quaternary salts and/or metal salts and/or ammonium salts and/or the resulting salts of 9- and/or 11-substituted apovincamic acid derivatives having the general formula I are transformed to the free 9- and/or 11-substituted apovincamic acid derivatives having the general formula I or to other acid additon salts and/or quaternary salts and/or metal salts and/or ammonium salts and/or the resulting racemic 9- and/or 11-substituted apovincamic acid derivatives having the general formula I and/or the acid addition salts and/or quaternary salts and/or metal salts and/or ammonium salts are dissociated to its or their optical antipodes and/or the corresponding optically active compounds are racemized to form stereo-isomers.

4. A process according to claim 3, characterized in that nitrating is effected with concentrated nitric acid in an acetic acid medium.

5. A process according to claim 3 or 4, characterized in that nitrating is effected in glacial acetic acid which contains 1 to 3% by volume acetonitrile or dimethyl formamide.

6. A process according to claim 3 or 4, characterized in that nitrating is effected in glacial acetic acid which contains 10 to 50% by volume chloroform.

7. A process according to claims 3 to 6, characterized in that nitrating is effected at temperatures from 0 to +16 °C.

8. A process according to claims 3 to 7, characterized in that the 9- and/or 11-(nitro)-apovincamic acid(s) is or are isolated as its or their nitrates.

9. A process according to claims 3 to 8, characterized in that the reduction is effected as a catalytic hydrogenation, particularly in the presence of palladium on activated carbon or Raney nickel, particularly at an approximately neutral pH value.

10. A process according to claims 3 to 8, characterized in that the reduction is effected by means of a chemical reducing agent, particularly tin in a hydrochloric acid medium.

**Claims for the Contracting State: AT**

1. A process of producing racemic or optically active 9- and/or 11-substituted apovincamic acid derivatives having the general formula

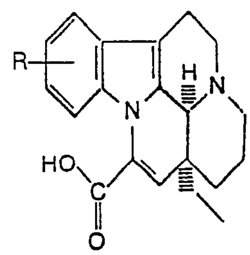

I

wherein

R is a nitro or amino group in position 9 or 11, and salts thereof, characterized in that apovincamic acid having the formula

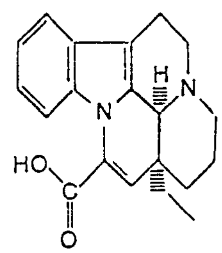

II

is nitrated and the resulting 9- and/or 11-(nitro)-apovincamic acid(s) is or are optionally reduced, optionally after it has or they have been isolated from the reaction mixture and, optionally the resulting 9- and/or 11-substituted apovincamic acid derivatives having the general formula I are transformed to acid addition salts or quaternary salts and/or metal salts and/or ammonium salts and/or the resulting salts of 9- and/or 11-substituted apovincamid acid derivatives having the general formula I are transformed to the free 9- and/or 11-substituted apovincamic acid derivatives having the general formula I or to other acid addition salts and/or quaternary salts and/or metal salts and/or ammonium salts and/or the resulting racemic 9- and/or 11-substituted apovincamic acid derivatives having the general formula I and/or the acid addition salts and/or quaternary and/or metal salts and/or ammonium salts are dissociated to its or their optical antipodes and/or the corresponding optically active compounds are racemized to form stereo-isomers.

2. A process according to claim 1, characterized in that nitrating is effected with concentrated nitric acid in an acetic acid medium.

3. A process according to claim 1 or 2, characterized in that nitrating is effected in glacial acetic acid which contains 1 to 3% by volume acetonitrile or dimethyl formamide.

4. A process according to claim 1 or 2, characterized in that nitrating is effected in glacial acetic acid which contains 10 to 50% by volume chloroform.

5. A process according to claims 1 to 4, characterized in that nitrating is effected at temperatures from 0 to +16 °C.

6. A process according to claims 1 to 5, characterized in that the 9- and/or 11-(nitro)-apovincamic acid(s) is or are isolated as its or their nitrates.

7. A process according to claims 1 to 6, characterized in that the reduction is effected as a catalytic hydrogenation, particularly in the presence of palladium on activated carbon or Raney nickel, particularly at an approximately neutral pH value.

8. A process according to claims 1 to 6, characterized in that the reduction is effected by means of a chemical reducing agent, particularly tin in a hydrochloric acid medium.

9. A process according to claims 1 to 8, characterized in that (+)-9-(nitro)-apovincamic acid, (+)-11-(nitro)-apovincamic acid, (−)-9-(amino)-apovincamic acid and (+)-11-aminoapovincamic acid and salts thereof are prepared.

**Revendications pour les Etats contractants: BE, CH, DE, FR, GB, IT, LI, NL, SE**

1. Dérivés de l'acide apovincaminique, racémiques ou optiquement actifs, substitués sur la position 9 et/ou sur la position 11, répondant à la formule générale

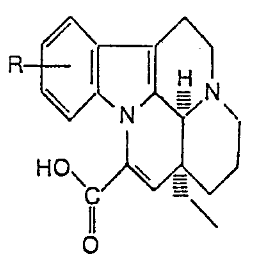

I

dans laquelle

R représente un groupe nitro ou amino en position 9 ou 11, ainsi que leurs sels.

2. Les dérivés de l'acide apovincaminique substitués sur la position 9 et/ou 11 suivants: l'acide (+)-9-(nitro)-apovincaminique, l'acide (+)-11-(nitro)-apovincaminique, l'acide (−)-9-(amino)-apovincaminique et l'acide (+)-11-aminoapovincaminique, ainsi que leurs sels.

3. Procédé de préparation des dérivés de l'acide apovincaminique substitués sur la position 9 et/ou 11 selon la revendication 1 ou 2, caractérisé en ce qu'on nitre de l'acide apovincaminique répondant à la formule

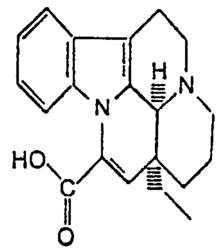

II

et l'on réduit éventuellement le ou les acide(s) 9- et/ou 11-(nitro)-apovincaminique(s) obtenus, éventuellement après leur isolement du mélange réactionnel, et éventuellement

l'on transforme les dérivés de l'acide apovincaminique substitués sur la position 9 et/ou 11, répondant à la formule générale I, obtenus en sels d'addition d'acides ou en sels quaternaires ou en sels de métaux ou d'ammonium et/ou l'on transforme les sels des dérivés de l'acide apovincaminique substitués sur la position 9 et/ou 11 répondant à la formule générale I obtenus en les dérivés libres de l'acide apovincaminique substitués sur la position 9 et/ou 11 répondant à la formule générale I, ou en d'autres sels d'addition d'acides ou sels quaternaires ou sels de métaux ou d'ammonium et/ou l'on effectue éventuellement une séparation des dérivés racémiques de l'acide apovincaminique substitués sur la position 9 et/ou sur la position 11 répondant à la formule générale I obtenus ou de leurs sels d'addition d'acides ou de leurs sels quaternaires ou de leurs sels de métaux ou d'ammonium en leurs antipodes optiques ou une racémisation en stéréo-isomères des composés optiquement actifs correspondants.

4. Procédé selon la revendication 3, caractérisé en ce qu'on effectue la nitration avec de l'acide nitrique concentré en milieu acétique glacial.

5. Procédé selon la revendication 3 ou 4, caractérisé en ce qu'on effectue la nitration dans de l'acide acétique glacial renfermant 1 à 3% en volume d'acétonitrile ou de diméthylformamide.

6. Procédé selon la revendication 3 ou 4, caractérisé en ce qu'on effectue la nitration dans de l'acide acétique glacial renfermant 10 à 50% en volume de chloroforme.

7. Procédé selon les revendications 3 à 6, caractérisé en ce qu'on effectue la nitration à des températures de 0 à +16 °C.

8. Procédé selon les revendications 3 à 7, caractérisé en ce qu'on effectue l'isolement du ou des acide(s) 9- et/ou 11-(nitro)-apovincaminique(s) sous la forme de leurs nitrates.

9. Procédé selon les revendications 3 à 8, caractérisé en ce qu'on effectue la réduction sous forme d'hydrogénation catalytique, en particulier en présence de palladium sur charbon actif ou nickel de Raney, surtout à un pH de valeur approximativement neutre.

10. Procédé selon les revendications 3 à 8, caractérisé en ce qu'on effectue la réduction avec un réducteur chimique, en particulier de l'étain en milieu chlorhydrique.

**Revendications pour l'Etat contractant: AT**

1. Procédé de préparation des dérivés de l'acide apovincaminique, racémiques ou optiquement actifs, substitués sur la position 9 et/ou sur la position 11, répondant à la formule générale

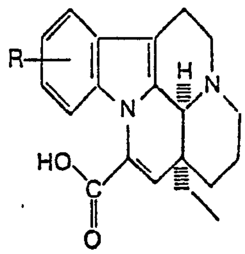

I.

dans laquelle

R représente un groupe nitro ou amino en position 9 ou 11, ainsi que leurs sels, caractérisé en ce qu'on nitre de l'acide apovincaminique répondant à la formule

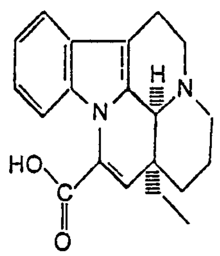

II

et l'on réduit éventuellement le ou les acide(s) 9- et/ou 11-(nitro)-apovincaminique(s) obtenus, éventuellement après leur isolement du mélange réactionnel, et éventuellement l'on transforme les dérivés de l'acide apovincaminique substitués sur la position 9 et/ou 11, répondant à la formule générale I, obtenus en sels d'addition d'acides ou en sels quaternaires ou en sels de métaux ou d'ammonium et/ou l'on transforme les sels des dérivés de l'acide apovincaminique substitués sur la position 9 et/ou 11 répondant à la formule générale I obtenus en les dérivés libres de l'acide apovincaminique substitués sur la position 9 et/ou 11 répondant à la formule générale I, ou en d'autres sels d'addition d'acides ou sels quaternaires ou sels de métaux ou d'ammonium et/ou l'on effectue éventuellement une séparation des dérivés racémiques de l'acide apovincaminique substitués sur la position 9 et/ou sur la position 11 répondant à la formule générale I obtenus ou de leurs sels d'addition d'acides ou de leurs sels quaternaires ou de leurs sels de métaux ou d'ammonium en leurs antipodes optiques ou une racémisation en stéréo-isomères des composés optiquement actifs correspondants.

2. Procédé selon la revendication 1, caractérisé en ce qu'on effectue la nitration avec de l'acide nitrique concentré en milieu acétique glacial.

3. Procédé selon la revendication 1 ou 2, caractérisé en ce qu'on effectue la nitration dans de l'acide acétique glacial renfermant 1 à 3% en volume d'acétonitrile ou de diméthylformamide.

4. Procédé selon la revendication 1 ou 2, caractérisé en ce qu'on effectue la nitration dans de l'acide acétique glacial renfermant 10 à 50% en volume de chloroforme.

5. Procédé selon les revendications 1 à 4, caractérisé en ce qu'on effectue la nitration à des températures de 0 à +16 °C.

6. Procédé selon les revendications 1 à 5, caractérisé en ce qu'on effectue l'isolement du ou des acide(s) 9- et/ou 11-(nitro)-apovincaminique(s) sous la forme de leurs nitrates.

7. Procédé selon les revendications 1 à 6, caractérisé en ce qu'on effectue la réduction sous la forme d'hydrogénation catalytique, en particulier en présence de palladium sur charbon actif ou nickel de Raney, surtout à un pH de valeur approximativement neutre.

8. Procédé selon les revendications 1 à 6, caractérisé en ce qu'on effectue la réduction avec un réducteur chimique, en particulier de l'étain en milieu chlorhydrique.

9. Procédé selon les revendications 1 à 8, caractérisé en ce que l'acide (+)-9-(nitro)-apovincaminique, l'acide (+)-11-(nitro)-apovincaminique, l'acide (−)-9-(amino)-apovincaminique et l'acide (+)-11-aminoapovincaminique, ainsi que leurs sels sont préparés.